Europäisches Patentamt

European Patent Office

Office européen des brevets

(19)

(11) Numéro de publication : **0 038 254 B1**

(12) **FASCICULE DE BREVET EUROPÉEN**

(45) Date de publication du fascicule du brevet :
18.01.84

(21) Numéro de dépôt : **81400561.7**

(22) Date de dépôt : **08.04.81**

(51) Int. Cl.³ : **A 01 J 15/12**, A 23 C 19/024, G 01 N 21/35, G 01 N 24/08, A 01 J 25/00

(54) **Procédé et installation pour mesurer et ajuster automatiquement en continu la teneur en eau, en matière grasse, en matières laitières non grasses (protéines et lactose), en sel, du beurre et des caillés de fromagerie.**

(30) Priorité : **11.04.80 FR 8008167**

(43) Date de publication de la demande :
**21.10.81 Bulletin 81/42**

(45) Mention de la délivrance du brevet :
**18.01.84 Bulletin 84/03**

(84) Etats contractants désignés :
**AT BE CH DE GB IT LI LU NL SE**

(56) Documents cités :
**DE-A- 2 358 307
DE-C-   863 165
FR-A- 2 050 525
FR-A- 2 087 203
FR-A- 2 178 803
FR-A- 2 273 279
US-A- 3 966 973**

(73) Titulaire : **TECNAL S.A. Société dite :
223, rue Jean-Jaurès
F-79001 Niort Cedex (FR)**

(72) Inventeur : **Lambrecht, Daniel
15, Impasse Pierre Puget
F-79000 Niort (FR)**

(74) Mandataire : **Rodhain, Claude
Cabinet Claude RODHAIN 30, rue La Boétie
F-75008 Paris (FR)**

Jouve, 18, rue St-Denis, 75001 Paris, France

Procédé et installation pour mesurer et ajuster automatiquement en continu la teneur en eau, en matière grasse, en matières laitières non grasses (protéines et lactose), en sel, du beurre et des caillés de fromagerie

L'invention concerne un procédé et une installation entièrement automatique permettant de contrôler, mesurer et réguler en conséquence la teneur en eau, en matière grasse, en matières laitières non grasses (protéines et lactose), en sel, du beurre et des caillés de fromagerie sortant d'une machine ou d'une chaîne de fabrication en continu.

Les législations sur la composition des produits alimentaires étant de plus en plus rigoureuses, et le fabricant étant par conséquent civilement et pénalement responsable de toute déclaration fausse ou mensongère pouvant induire en erreur le consommateur, on est contraint de doter les machines de fabrication d'organes de contrôle et de mesurage permettant de détecter à tout moment un déséquilibre de la proportion des constituants dans le mélange, afin d'agir rapidement sur ces constituants pour les réguler en conséquence, de manière à obtenir l'harmonie recherchée.

Or la difficulté est que la détection et la régulation doivent se faire dans un temps de réponse très court, compte tenu du débit des machines travaillant en continu.

Certains fabricants ont tenté de trouver des solutions applicables au beurre et aux caillés de fromagerie, sans toutefois y parvenir. C'est ainsi que l'on a proposé, dans le domaine du beurre (ou la teneur en eau légale ne doit pas dépasser 16 %), de mesurer et régler en continu la teneur en eau. Mais, là encore, cette solution s'est avérée infructueuse en raison du fait que la mesure continue devenait fausse dès que la composition de la crème changeait et dès que l'on modifiait le réglage d'un des organes de la baratte continue ou de sa pompe d'alimentation en crème. Pour remédier à cet inconvénient, il fallait alors faire une analyse de la teneur en eau en laboratoire et ré-étalonner l'appareil de mesure en continu, en accord avec le résultat de l'analyse de laboratoire. Or, sachant qu'il faut au moins dix minutes pour avoir le résultat de l'analyse en laboratoire, la baratte continuait à fabriquer pendant ce temps une quantité de beurre qui pouvait atteindre une tonne et dont la composition était éventuellement illégale.

Une autre solution connue devait également permettre de mesurer et de régler la teneur en eau du beurre. Ce procédé consistait à évaporer en continu l'humidité contenue dans 1 ‰ (un pour mille) de la production de beurre et par pesée différentielle avant et après évaporation, à obtenir une mesure de la teneur en eau du beurre. Le matériel correspondant à ce procédé n'a jamais été commercialisé car la précision obtenue était insuffisante.

Plus récemment, des analyseurs rapides utilisant le principe de l'absorption infrarouge ont apporté une solution parfaite pour la mesure et la régulation de la teneur en matière grasse du lait standard fabriqué en continu. La solution a été possible parce que ces analyseurs donnent un résultat d'analyse parfaitement précis dans un temps très court, de l'ordre de 10 à 30 secondes, et que cette mesure d'analyse peut être exploitée sans inconvénient par rapport à une mesure continue. Toutefois, l'analyseur précédemment cité n'a pu être exploité que parce que le lait standard étant liquide, il était facile de prélever un échantillon et de l'introduire automatiquement dans la cellule de mesure de l'analyseur, comme décrit par exemple dans FR-A-2 050 525.

On a également utilisé un analyseur à résonance magnétique nucléaire pour déterminer en continu la teneur en eau d'un produit pâteux comme décrit dans US-A-3 966 973, en utilisant les résultats de la mesure pour régler la teneur en eau.

On connaît enfin un procédé et un dispositif décrits dans FR-A-2 087 203 pour mesurer en continu la teneur en eau du beurre, suivant lesquels on dérive sous pression une partie aliquote du débit de beurre à la sortie du butyrateur et on effectue une mesure différentielle continue sur cette partie, dont on exploite les résultats.

Cette partie aliquote est prélevée au moyen d'un orifice calibré du butyrateur et pompée sous pression dans un appareil comprenant deux cellules de mesure en amont et en aval, d'un réchauffeur et d'un évaporateur qui sépare l'extrait sec. Une pompe de reprise renvoie l'extrait sec de la première cellule à la seconde, puis la partie aliquote mesurée est alors évacuée.

Ce procédé et ce dispositif ne permettent pas d'effectuer des mesures dans un appareil d'analyse par réflexion infrarouge ou résonance magnétique nucléaire, du fait que dans un tel appareil l'échantillon à mesurer doit séjourner un laps de temps minimum pour effectuer la mesure, ce qui nécessiterait l'arrêt du prélèvement à chaque mesure et la remise en marche nécessitant un nouveau temps de fonctionnement pour vider les conduits du dispositif et amener une nouvelle partie aliquote prélevée dans le butyrateur, afin d'éviter d'effectuer la mesure suivante sur du beurre se trouvant déjà contenu dans les conduits au moment de la première mesure.

Il est donc nécessaire de prélever la partie aliquote du beurre dans un circuit dans lequel celui-ci est constamment renouvelé et de façon telle qu'on soit assuré qu'à la fin de chaque mesure le beurre contenu dans les conduits ait été évacué et qu'une quantité de beurre différente est immédiatement disponible au voisinage de l'appareil d'analyse pour effectuer la mesure suivante.

Le but de l'invention est de résoudre ce problème.

La présente invention concerne donc un procédé et une installation exploitant les capacités des analyseurs connus utilisant le principe de la réflexion infrarouge ou de l'absorption infrarouge ou même de la résonance magnétique nucléaire dans le domaine nouveau du beurre et des caillés de fromagerie, c'est-à-dire de produits pâteux, en vue de mesurer et de réguler entièrement automatiquement et en continu leur teneur en eau, en matière grasse, en matières laitières non grasses (protéines et lactose) ou encore en sel.

L'invention concerne plus particulièrement un procédé pour mesurer et ajuster automatiquement en continu la teneur en eau, en matière grasse, en matières laitières non grasses (protéines et lactose), et en sel, du beurre et du caillé de fromagerie débité en continu par une machine de fabrication, le mesurage étant réalisé automatiquement, au moyen d'un appareil analyseur connu en soi utilisant notamment le principe de la réflexion ou de l'absorption infrarouge ou la résonance magnétique nucléaire, sur une partie aliquote du beurre prélevée automatiquement par pompage et dans lequel on utilise la sortie de l'appareil d'analyse pour commander la composition du beurre, procédé caractérisé en ce que l'on prélève ladite partie aliquote dans un circuit de dérivation fermé sur la machine de fabrication et comprenant une première pompe de recyclage, on liquéfie ladite partie aliquote par chauffage et on la fait ensuite passer à l'aide d'une seconde pompe dans un dispositif comprenant des moyens pour transférer un échantillon de ladite partie aliquote dans ledit appareil d'analyse, et on effectue la mesure sur ledit échantillon pendant qu'on recycle dans la machine, en circuit fermé, le reste de ladite partie aliquote au moyen de ladite seconde pompe, et on règle les proportions des constituants comme connu en soi au moyen de la sortie de l'appareil d'analyse en utilisant des conduits distincts pour les différents constituants.

L'invention est également caractérisée par le fait qu'un échantillon prélevé automatiquement dans un circuit en boucle fermée par la machine de fabrication en continu est introduit automatiquement par pompage dans la cellule de mesure de l'appareil analyseur et est seulement ramolli ou liquéfié ou bien est en plus dilué automatiquement. La liquéfaction de l'échantillon peut être effectuée par fusion avant ou après dilution éventuelle, cette dernière pouvant être réalisée à partir d'eau pure ou d'un autre diluant dont la quantité est proportionnelle à celle de l'échantillon.

L'invention s'étend également à une installation pour la mise en œuvre de ce procédé, caractérisée essentiellement en ce qu'elle comprend un premier circuit de dérivation comportant une première pompe de recyclage et formant une boucle fermée sur la machine de fabrication, une liaison de ce premier circuit avec une seconde pompe par l'intermédiaire de moyens de liquéfaction par chauffage, puis, à la suite de cette seconde pompe un dispositif comprenant des moyens pour transférer un échantillon de la partie aliquote prélevée à l'appareil analyseur et des moyens pour recycler le reste de ladite partie aliquote dans la machine de fabrication, formant ainsi un deuxième circuit de dérivation, et enfin sur la sortie de l'appareil analyseur des moyens de régulation distincts pour agir sur les proportions des constituants du produit fabriqué.

D'autres caractéristiques et avantages ressortiront de la description ci-après et des deux dessins annexés et qui sont des vues schématiques de l'installation vue selon deux variantes de réalisation.

Comme indiqué précédemment, l'originalité de l'invention repose sur un procédé et une installation permettant de mesurer et de réguler automatiquement en continu les composants du beurre et des caillés de fromagerie fabriqués dans une chaîne travaillant en continu.

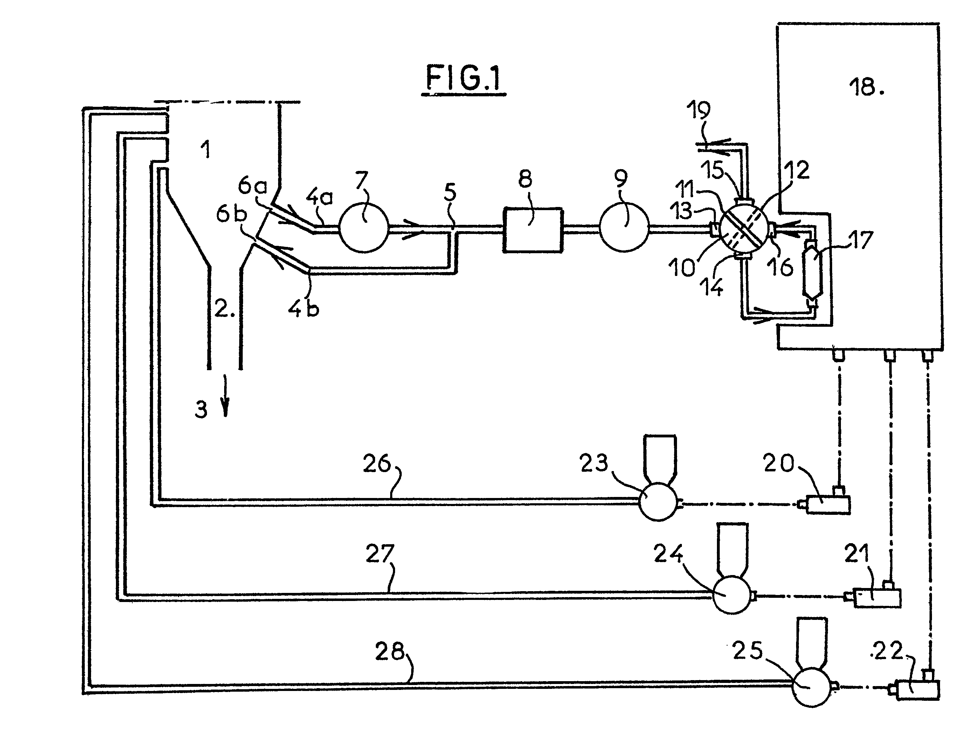

La figure 1 annexée représente la buse de sortie 1 de la machine à beurre de laquelle sort le ruban de beurre 2 en direction de la flèche 3. Cette buse comporte deux orifices supplémentaires, un orifice 6a par lequel est canalisé du beurre dans un circuit parallèle 4a en dérivation, ce circuit reconduisant le beurre dans la buse par l'orifice 6b. Afin de renouveler le beurre suffisamment vite dans le circuit parallèle 4a-4b, une pompe 7 est prévue, celle-ci permettant une circulation accélérée du beurre afin de le rendre plus homogène et par conséquent plus représentatif en vue de la prise d'échantillon. Sur la canalisation 4a est prévu un orifice 5 par lequel une très faible quantité de beurre est dérivée et ramollie ou fondue par un élément chauffant thermostaté 8. Une pompe 9 règle le débit du beurre ramolli ou fondu dans tout le circuit compris entre l'orifice 5 et une conduite de recyclage 19. En sortant de la pompe 9, le beurre pénètre par l'orifice 13 d'un distributeur à palette 10 et lorsque le tiroir est en position 11, il en ressort par l'orifice 14, traverse ensuite la capsule de mesure 17 de l'analyseur 18, repénètre par 16 dans le distributeur 10 et en ressort par 15 en direction de la conduite de recyclage 19. Au bout de quelques secondes, une minuterie déplace le tiroir du distributeur de la position 11 à la position 12, ce qui arrête la circulation du beurre dans la capsule de mesure 17 puisque les orifices 14 et 16 se trouvent isolés et provoquent la mise en service de l'analyseur 18. Pendant que le tiroir est en position 12, le beurre continue à circuler mais en allant directement de l'orifice d'entrée 13 à l'orifice de sortie 15 du distributeur puis en prenant la conduite de recyclage 19. Dès que l'analyseur 18 a donné les résultats des analyses et arrête son propre processus interne, un système de relayage électrique connu replace le tiroir du distributeur en position 11 et le cycle déjà décrit recommence. Il est à noter que, d'une part, tous les circuits sont hermétiquement étanches de sorte que le produit soit à l'abri de l'air et que, d'autre part, le contenu de la capsule de l'analyseur est renouvelé par la circulation hermétique créée par les moyens de prélèvement et de pompage pendant le laps de temps où ce contenu n'est pas soumis à l'action de l'analyseur. En outre, le produit pénètre dans la capsule de mesure par le bas et en ressort par le haut de sorte qu'il n'existe aucune canalisation descendante à partir du haut de la capsule, ceci afin que les bulles d'air éventuelles puissent se dégager librement et sortir par le haut de la capsule.

L'analyseur, connu en soi, est capable de mesurer la teneur en matière grasse, la teneur en matières laitières non grasses (protéines, lactose) et encore la teneur en eau ou en matières sèches. De même, cet analyseur est capable de mesurer la teneur en sel ou tout au moins d'indiquer cette teneur par différence entre le total des matières sèches diminué de la somme : matière grasse, plus protéines, plus lactose. S'agissant du beurre, cette installation peut permettre de mesurer et de réguler en continu les composants : eau, matières non grasses, et sel dans le cas de beurre salé. S'agissant d'un caillé de fromagerie, elle permet de mesurer et de réguler en continu les composants : matière grasse, matière non grasse et sel.

Les résultats indiqués par l'analyseur 18 apparaissant respectivement en 20, 21 et 22 commandent l'entrée en action de doseurs d'appoint 23, 24 et 25 ayant pour vocation d'augmenter séparément la teneur de chacun des constituants, dans des proportions différentes de manière à ré-équilibrer la composition prescrite du produit. Ces doseurs d'appoint sont montés en parallèle sur des circuits 26, 27 et 28 menant à la buse de sortie 1.

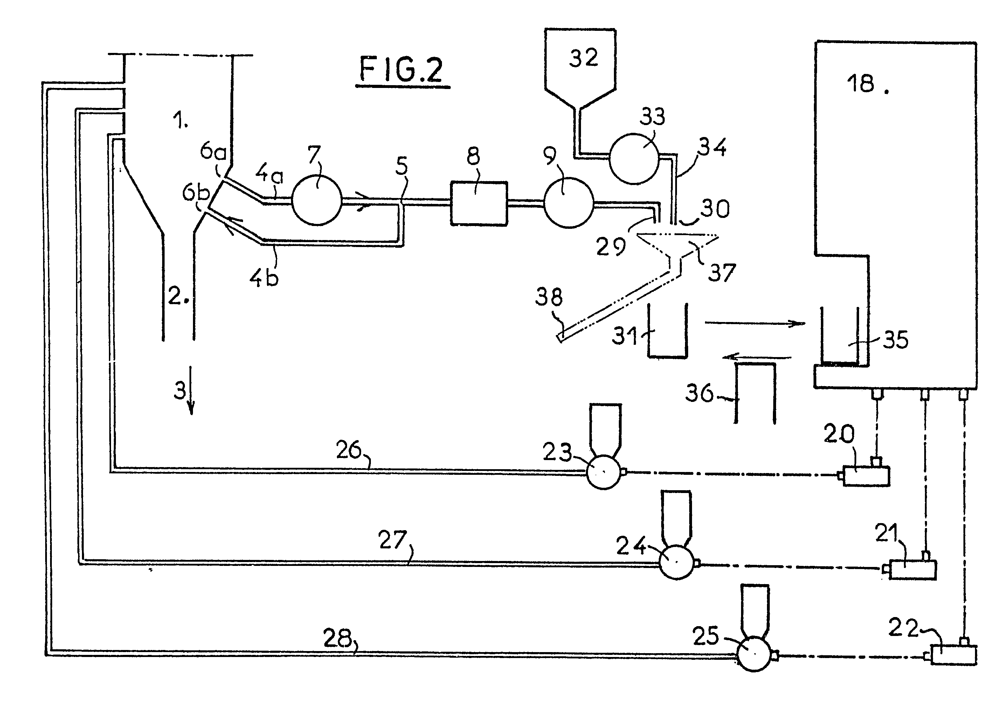

La figure 2 représente une variante de l'invention caractérisée en ce que l'analyseur, connu en soi, et fonctionnant selon les principes précédemment décrits est d'un modèle non capable d'analyser le beurre ou les caillés de fromagerie à l'état pur parce que leur teneur en matière grasse et en matières non grasses est trop élevée et qu'il sera seulement en mesure d'effectuer les analyses après dilution obligatoire de l'échantillon. De plus, ce type d'analyseur connu est pourvu d'une capsule ou gobelet (31) de mesure mobile de type ouvert. Le fonctionnement selon la figure 2 est identique à celui de la figure 1 jusqu'à l'arrivée du beurre fondu dans la pompe 9 qui règle le débit du beurre fondu dans tout le circuit compris entre l'orifice 5 et un bec d'écoulement 29. Parallèlement à ce bec verseur 29 et à proximité de ce dernier, est prévu un second bec verseur 30 venant déverser dans le gobelet 31 une quantité d'eau pure ou de diluant proportionnelle au débit de produit liquéfié déversé par le bec 29. Ces moyens de dilutions sont situés à proximité des moyens de liquéfaction.

Ce bec verseur 30 est alimenté à partir d'une réserve en eau 32 par le jeu d'une pompe 33 située sur le conduit 34 menant à ce bec verseur 30. Ainsi, et par cette disposition, on dilue le produit liquéfié par l'organe de chauffage 8, par adjonction d'eau déversée selon une quantité déterminée dans le gobelet recevant le produit liquéfié.

Le mélange de produit liquéfié et d'eau est ensuite automatiquement transféré depuis la position du gobelet 31 à la position 35 de manière à amener ledit mélange dans l'enceinte de l'analyseur 18 connu en soi. Ce transfert est obtenu par le fait que le gobelet 31 est animé d'un mouvement de translation alternatif, de façon à pouvoir prendre les deux positions illustrées par les références 31 et 35. Ce gobelet est non seulement mobile en translation, mais est également monté pivotant, de façon à pouvoir être renversé dans la position intermédiaire 36, pour déverser le reliquat du mélange après analyse.

Bien que les moyens permettant le déplacement en translation et en rotation du gobelet ne soient pas représentés, on conçoit que ceux-ci peuvent être constitués de façon connue d'un vérin ou d'un petit convoyeur. Afin d'éviter le refroidissement du mélange pendant son transfert en direction de l'analyseur, le gobelet peut être thermostaté afin de maintenir à température constante le mélange liquéfié et dilué.

Les résultats indiqués par l'analyseur 18 apparaissant respectivement en 20, 21 et 22 commandant l'entrée en action des doseurs d'appoint 23, 24 et 25 ayant pour vocation d'augmenter séparément la teneur de chacun des constituants, dans des proportions différentes de manière à ré-équilibrer la composition prescrite du produit. Ces doseurs d'appoint sont montés en parallèle sur des circuits 26, 27 et 28 menant à la buse de sortie 1.

Ainsi les résultats de l'analyseur sont exploités immédiatement et en continu pour réguler automatiquement la proportion des constituants du beurre ou du caillé de fromagerie, le temps de réponse entre l'émission des résultats de l'analyseur et son intervention pour modifier la proportion desdits constituants étant extrêmement court, ce qui est un avantage fondamental lorsqu'on sait que les machines de fabrication en continu débitent six tonnes à l'heure. On a observé que les analyses peuvent avoir lieu toutes les 10 ou 30 secondes, de sorte que le contrôle est parfaitement assuré par une fréquence des réglages suffisamment rapprochés pour avoir la certitude de fabriquer un produit dans des conditions optimum de composition.

L'avantage de cette installation réside par ailleurs dans le fait que le prélèvement du produit est automatique et que le fonctionnement de tous les organes de mesurage, de contrôle, de transfert sont automatisés, ce qui permet précisément de réduire au minimum le laps de temps s'écoulant entre le prélèvement de l'échantillonnage et le dosage des composants à la suite du travail de l'analyseur et d'éviter toute intervention manuelle dans le déroulement des opérations.

La partie aliquote peut être prélevée en continu ou de façon discontinue, mais dans le cas de prélèvement ininterrompu, on prévoira dans le cas de la figure 2, sous les becs verseurs 29 et 30, un entonnoir de recyclage 37 permettant de réintroduire en continu, par le conduit 38, la partie aliquote dans la buse de sortie 1.

Bien entendu, on pourra faire varier l'ordonnancement des opérations du procédé sans pour cela sortir du cadre de l'invention.

**Revendications**

1. Procédé pour mesurer et ajuster automatiquement en continu la teneur en eau, en matières grasses, en matières laitières non grasses (protéines et lactoses), et en sel du beurre et du caillé de fromagerie débité en continu par une machine de fabrication, le mesurage étant réalisé automatiquement, au moyen d'un appareil analyseur (18) connu en soi utilisant notamment le principe de la réflexion ou de l'absorption infra-rouge ou la résonance magnétique nucléaire, sur une partie aliquote du beurre prélevée automatiquement par pompage et dans lequel on utilise la sortie de l'appareil d'analyse (18) pour commander la composition du beurre, procédé caractérisé en ce qu'on prélève ladite partie aliquote dans un circuit de dérivation (6a-4a-6b) fermé sur la machine de fabrication et comprenant une première pompe (7) de recyclage, on liquéfie ladite partie aliquote par chauffage, et on la fait ensuite passer à l'aide d'une seconde pompe (9) dans un dispositif comprenant des moyens pour transférer un échantillon de ladite partie aliquote dans ledit appareil d'analyse (18), et on effectue la mesure sur ledit échantillon pendant qu'on recycle dans la machine, en circuit fermé (19 ; 37, 38), le reste de ladite partie aliquote, au moyen de ladite seconde pompe (9), et on règle les proportions des constituants comme connu en soi au moyen de la sortie (20-22) de l'appareil d'analyse (18) en utilisant des conduits distincts (26-28) pour les différents constituants.

2. Procédé selon la revendication 1, caractérisé en ce qu'on liquéfie et on dilue l'échantillon avant son transfert dans l'appareil analyseur (18).

3. Procédé selon l'une quelconque des revendications 1 et 2, caractérisé en ce qu'on liquéfie l'échantillon avant ou après sa dilution.

4. Procédé selon la revendication 3, caractérisé en ce que l'on réalise la dilution par apport d'eau pure dont la quantité est proportionnelle à celle de l'échantillon.

5. Procédé selon l'une quelconque des revendications 1 à 4, caractérisé en ce que les circuits (6a-4a-6b ; 19) où sont effectués le prélèvement, le pompage, le transfert de l'échantillon dans l'appareil analyseur et le recyclage, sont hermétiquement étanches vis-à-vis de l'extérieur.

6. Procédé selon l'une quelconque des revendications 1 à 5, caractérisé en ce qu'on renouvelle le contenu de la capsule (17), recevant l'échantillon, de l'appareil analyseur (18) au moyen d'un circuit hermétique, vis-à-vis de l'extérieur pendant le laps de temps où ce contenu n'est pas soumis à l'action de l'appareil analyseur.

7. Installation pour la mise en œuvre du procédé selon l'une quelconque des revendications 1 à 6, caractérisée en ce qu'elle comprend un premier circuit de dérivation (4a, 4b) comportant une première pompe (7) de recyclage et formant une boucle fermée sur la machine de fabrication, une liaison (5) de ce premier circuit (4a, 4b) avec une seconde pompe (9) par l'intermédiaire de moyens (8) de liquéfaction par chauffage, puis, à la sortie de cette seconde pompe (9) un dispositif comprenant des moyens (10, 31) pour transférer un échantillon de la partie aliquote prélevée à l'appareil analyseur (18) et des moyens (19, 37) pour recycler le reste de ladite partie aliquote dans la machine de fabrication, formant ainsi un deuxième circuit de dérivation, et enfin sur la sortie de l'appareil analyseur (18), des moyens de régulation distincts (23, 24, 25) pour agir sur les proportions des constituants du produit fabriqué.

8. Installation selon la revendication 7, caractérisée en ce qu'elle comprend des moyens de dilution (32, 33) disposés en aval de la seconde pompe (9).

9. Installation selon la revendication 8, caractérisée en ce que les moyens de dilution sont constitués d'une réserve d'eau pure (32) et d'une pompe (33) situées en aval desdits moyens de liquéfaction (8) mais à proximité de ceux-ci pour que le produit fondu et le produit de dilution soient déversés dans les moyens de transfert (31) dudit dispositif.

10. Installation selon la revendication 9, caractérisée en ce que les moyens de transfert sont constitués d'un gobelet (31), servant de mesure de capacité, animé d'un mouvement de translation alternative depuis un poste d'alimentation jusqu'au poste (35) de l'appareil analyseur (18).

11. Installation selon la revendication 10, caractérisée en ce que le gobelet (31) est monté basculant pour pouvoir être retourné.

12. Installation selon la revendication 7, caractérisée en ce que ledit dispositif de transfert d'un échantillon et de recyclage du reste de la partie aliquote comprend un distributeur (10) adapté pour relier automatiquement la sortie de la seconde pompe (9) à une capsule de mesure (17) située dans l'appareil analyseur (18) et à des moyens de recyclage (19), alternativement.

**Claims**

1. Method for the continuous automatic measurement and adjustment of the water content, fat content, non-fatty milk substance content (proteins and lactoses) and salt content of butter and of curds from the cheese-making industry, discharged continuously by a production machine, the measurement taking place automatically by means of an analysis apparatus (18) known *per se*, using, in particular the principle of infrared reflection or absorption or nuclear magnetic resonance on an aliquot part of the butter taken automatically by means of pumping, and in which the outlet of the analysis apparatus (18) is used to control the composition of the butter, characterized in that the said aliquot part is taken from a closed by-pass circuit (6a-4a-6b) which is on the production machine and comprises a first recycling pump (7), the said aliquot part is liquefied by heating, and is subsequently made to pass by means of a second pump (9) into a device comprising means for transferring a sample of the said aliquot part into the said analysis apparatus (18), and the sample is measured whilst the rest of the said aliquot part is recycled in the machine, in a closed circuit (29, 37, 38), by means of the said second pump (9), and the proportions of the constituents are controlled as known *per se* by means of the outlet (20-22) of the analysis apparatus (18), using different pipes (26-28) for the various constituents.

2. Method according to claim 1, characterized in that the sample is liquefied and diluted before being transferred into the analysis apparatus (18).

3. Method according to either claim 1 or 2, characterized in that the sample is liquefied before or after it has been diluted.

4. Method according to claim 3, characterized in that dilution is carried out by adding pure water in a quantity proportional to that of the sample.

5. Method according to any one of claims 1 to 4, characterized in that the circuits (6a-4a-6b ; 19) where the sampling, pumping, transfer of the sample into the analysis apparatus and recycling are carried out, are hermetically sealed with respect to the outside.

6. Method according to any one of claims 1 to 5, characterized in that the content of the capsule (17) — which accomodates the sample — of the analysis apparatus (18) is renewed by way of a circuit hermetically sealed with respect to the outside during the period of time when this content is not subject to the action of the analysis apparatus.

7. Installation for carrying out the method according to any one of claims 1 to 6, characterized in that it comprises a first by-pass circuit (4a, 4b) having a first recycling pump (7) and forming a closed loop on the production machine, a connexion (5) between this first circuit (4a, 4b) and a second pump (9) by way of means (8) of liquefaction by heating, then at the outlet of this second pump (9) a device comprising means (10, 31) for transferring a sample of the aliquot part taken from the analysis apparatus (18) and means (19, 37) for recycling the rest of the said aliquot part in the production machine, thus forming à second by-pass circuit, and finally, on the outlet of the analysis apparatus (18), different control means (23, 24, 25) for influencing the proportions of the constituents of the manufactured product.

8. Installation according to claim 7, characterized in that it comprises dilution means (32, 33) disposed downstream from the second pump (9).

9. Installation according to claim 8, characterized in that the dilution means consist of a reserve (32) of pure water and a pump (33) situated downstream from the said liquefaction means (8) but close to the latter so that the melted product and the product of dilution can be poured into the transfer means (31) of the said device.

10. Installation according to claim 9, characterized in that the transfer means consist of a container (31) serving as a measure of the capacity, displaced in a reciprocating manner from a feed station to a station (35) of the analysis apparatus (18).

11. Installation according to claim 10, characterized in that the container (31) is mounted in a rocking manner so that it can be turned over.

12. Installation according to claim 7, characterized in that the said device for transferring a sample and for recycling the rest of the aliquot part comprises a distributor (10) adapted to automatically connect the outlet of the second pump (9) alternately to a measuring capsule (17) situated in the analysis apparatus (18) and to recycling means (19).

**Ansprüche**

1. Verfahren zum kontinuierlichen und automatischen Messen und Einstellen des Gehaltes an Wasser-, Fett-, Nichtfett-Milchstoffen (Proteinen und Laktose) und des Salzgehaltes von kontinuierlich einer Fabrikationsmaschine entnommener Butter und Käsebruch, wobei das Messen eines durch Pumpen automatisch entnommenen aliquoten Teiles der Butter durch einen an sich bekannten Analyseapparat automatisch erfolgt, welcher insbesondere auf dem Prinzip der Reflexion oder der Absorption von Infrarotstrahlen oder der nuklearen Magnetresonanz arbeitet, bei welchem Meßvorgang die Zusammensetzung der Butter am Ausgang des Analyseapparates (18) beeinflußt wird, dadurch gekennzeichnet, daß der genannte aliquote Teil aus einem abgeschlossenen Ablenkkreis (6a-4a-6b) der Fabrikationsmaschine entnommen wird, der eine erste Recyclingpumpe (7) enthält, daß der entnommene aliquote Teil durch Erhitzen verflüssigt wird, daß er anschließend mit Hilfe einer zweiten Pumpe (9) in ein Gerät mit Einrichtungen zum Transportieren einer Probe des genannten aliquoten Teiles in den genannten Analyseapparat (18) gebracht wird, daß die Messung der genannten Probe vorgenommen wird, während im geschlossenen Kreis (19 ; 37, 38) der Rest des genannten aliquoten Teiles mit Hilfe der genannten zweiten Pumpe (9) in die Maschine rückgeführt wird, und daß das Verhältnis der Bestandteile in an sich bekannter Weise am Ausgang (20-22) des Analyseapparates (18) unter Benutzung unterschiedlicher Leitungen (26-28) für die verschiedenen Bestandteile reguliert wird.

2. Verfahren nach Anspruch 1, dadurch gekennzeichnet, daß die Probe vor ihrem Transport in den Analyseapparat (18) verflüssigt und verdünnt wird.

3. Verfahren nach einem der Ansprüche 1 und 2, dadurch gekennzeichnet, daß die Probe vor oder nach ihrer Verdünnung verflüssigt wird.

4. Verfahren nach Anspruch 3, dadurch gekennzeichnet, daß die Verdünnung durch Zugabe von reinem Wasser vorgenommen wird, das in einer der Menge der Probe proportionalen Menge zugegeben wird.

5. Verfahren nach einem der Ansprüche 1 bis 4, dadurch gekennzeichnet, daß die Leitungen (6a-4a-6b ; 19), in denen das Entnehmen, das Pumpen und der Transport der Probe in den Analyseapparat und die Rückführung erfolgen, hermetisch gegen die Umgebung abgedichtet sind.

6. Verfahren nach einem der Ansprüche 1 bis 5, dadurch gekennzeichnet, daß der Inhalt der die Probe aufnehmenden Kapsel (17) des Analyseapparates (18) mittels einer hermetisch gegen die Umgebung abgedichteten Leitung während jener Zeitspanne erneuert wird, während der der Inhalt nicht durch den Analyseapparat geprüft wird.

7. Vorrichtung zur Durchführung des Verfahrens nach einem der Ansprüche 1 bis 6, dadurch gekennzeichnet, daß sie einen ersten Ablenkkreis (4a, 4b) mit einer ersten Recyclingpumpe (7) umfaßt, der eine erste geschlossene Schleife an der Fabrikationsmaschine bildet, eine Verbindung (5) dieses ersten Kreises (4a, 4b) mit einer zweiten Pumpe (9) unter Zwischenschaltung von Verflüssigungseinrichtungen (8) durch Erhitzen, und dann am Ausgang dieser zweiten Pumpe (9) eine Einrichtung aufweist, die Einrichtungen (10, 31) zum Transport der Probe aus dem entnommenen aliquoten Teil zum Analyseapparat (18) sowie Einrichtungen (19, 37) zum Rückführen des Restes des genannten aliquoten Teiles in die Fabrikationsmaschine, welche somit einen zweiten Ablenkkreis bilden, und schließlich am Ausgang des Analyseapparates (18) unterschiedliche Reguliereinrichtungen (23, 24, 25), um auf das Verhältnis der Bestandteile des erzeugten Produktes einwirken zu können.

8. Vorrichtung nach Anspruch 7, dadurch gekennzeichnet, daß sie nach der zweiten Pumpe (9) angeordnete Verdünnungseinrichtungen (32, 33) aufweist.

9. Vorrichtung nach Anspruch 8, dadurch gekennzeichnet, daß die Verdünnungseinrichtungen aus einem Reservoir (32) mit reinem Wasser und aus einer nach aber in der Nähe der genannten Verflüssigungseinrichtung (8) angeordneten Pumpe (33) bestehen, damit das geschmolzene und das Verdünnungsprodukt in die Transporteinrichtung (31) der genannten Einrichtung fließen.

10. Vorrichtung nach Anspruch 9, dadurch gekennzeichnet, daß die Transporteinrichtungen aus einem Becher (31) bestehen, der die Menge mißt und der durch eine hin- und hergehende Verschiebung von der Einfüllstelle bis zur Untersuchungsstelle (35) des Analyseapparates (18) bewegt wird.

11. Vorrichtung nach Anspruch 10, dadurch gekennzeichnet, daß der Becher (31) schwenkbar befestigt ist, damit er umgedreht werden kann.

12. Vorrichtung nach Anspruch 7, dadurch gekennzeichnet, daß die genannte Einrichtung zum Transport einer Probe und zur Rückführung des Restes des aliquoten Teiles einen Verteiler (10) aufweist, der den Ausgang der zweiten Pumpe (9) automatisch abwechselnd mit einer im Analyseapparat (18) vorgesehenen Meßkapsel (17) und mit Rückführungseinrichtungen (19) verbindet.

FIG.1

FIG.2